# EUROPEAN PATENT SPECIFICATION

(11) **EP 2 389 115 B1**
(45) Date of publication and mention of the grant of the patent: **28.02.2018**
(21) Application number: 09833888.2
(22) Date of filing: 21.12.2009
(51) Int. Cl.: A61B 10/00, A61B 10/02, A61B 10/04

(54) **NAVIGABLE TISSUE TREATMENT TOOLS**
NAVIGIERBARE GEWEBEBEHANDLUNGSWERKZEUGE
OUTILS DE TRAITEMENT TISSULAIRE POUVANT NAVIGUER

(30) Priority: 19.12.2008 US 139501 P
(43) Date of publication of application: 30.11.2011
(73) Proprietor: Covidien LP, Mansfield, MA 02048 (US)
(72) Inventor: JOHNSON, Jay, Plymouth MN 55441-5433 (US); BORILLO, Tom, Plymouth MN 55441-5433 (US); HASTINGS, Paige, Bloomington MN 55437 (US); MCKINLEY, David, J., Chanhassen MN 55317 (US)
(74) Representative: Soames, Candida Jane
(86) International application number: PCT/US2009/069073
(87) International publication number: WO 2010/071895

(56) References cited:
- WO-A2-2008/023193
- US-A1- 2001 007 925
- US-A1- 2002 123 697
- US-A1- 2003 050 572
- US-A1- 2004 260 201
- US-A1- 2004 260 201

## Description

### BACKGROUND OF THE INVENTION

It is known to use tools to take tissue samples. For example, US 2004/260201 discloses a cytology brush, sized for entry into a mammary duct of a human patient and comprising: a bristle shank; a bristle structure on the shank and having bristles of sufficient stiffness to loosen cytology material lining the mammary duct without substantial traumatization of mammary duct tissue; a brush handle; and a coupling for releasably attaching the bristle shank to the brush handle.
WO 2008/023193 is considered the closest prior art to the subject-matter of claim 1 and discloses a medical device for sampling biological material comprising; an elongate housing; a probe for sampling the biological material mounted within the elongate housing; and a current supply electrically coupled to the probe or the elongate housing; wherein said elongate housing and said probe are made from materials having different Peltier potentials such that supply of current from the current supply generates a Peltier effect between said elongate housing and said probe. A tip of the probe may be removable therefrom.

Identifying and treating lung tissue abnormalities presents challenges that are somewhat unique to the lungs. If a tissue lesion or tumor is to be identified and excised surgically, the chest wall must be opened to provide access to the lungs. Opening the chest wall is a common procedure but one that presents risks of infection and lengthy recovery time, nonetheless. If a tissue lesion or tumor is to be identified endoscopically, the complicated bronchial maze must be navigated.

Technology has been developed that allows a physician to track, in real-time, the position of a probe (hereinafter "locatable guide" or "LG") traveling through the airways. This technology incorporates a plurality of coils at the end of an LG and a magnetic field generator outside of the patient. The patient is placed in the magnetic field created by the generator. As the LG is navigated through the airways, electrical current is induced in the coils and sent via conductors to a computer. The computer can calculate the position and orientation of the probe based on the relative strengths of the current being induced. This technology is shown and described in greater detail in U.S. Patents 7,233,820 6,226,543, 6,188,355, 6,380,732, 6,593,884, 6,711,429, 6,558,333, 6,887,236, 6,615,155, 6,574,498, 6,947,788, 6,996,430, 6,702,780, and 6,833,814; and U.S. Patent Publications 20050171508, 20030074011, 20020193686, and also PCT application WO 03/086498 titled "Endoscope Structure and Techniques for Navigation in Branched Structure" to Giboa.

One type of tool used with the above-described system is a biopsy tool. Biopsy tools are designed to remove and retrieve small tissue samples from a suspected lesion for analysis and identification in a laboratory. Several factors are considered when taking biopsies of tissue such as biopsy location, biopsy size, and the number of samples needed from a suspected lesion.

The above system and apparatus are aimed at the first consideration, target location, and provide a system that enables a physician to navigate standard bronchoscopic tools, such as biopsy tools, to a target located in the lung. In its basic operation, the target is first identified in the CT data, and then the locatable guide is navigated to the target. The locatable guide is then removed from a sheath surrounding the LG and the sheath is then used as an extended working channel ("EWC") through which a biopsy tool may be passed to the target location.

Once the LG is removed, however, the locating system is no longer useful in identifying the location of the tip of the EWC. Hence, with regard to the biopsy tool, the physician is effectively "operating in the blind." Additionally, quite often it is desired to take multiple samples from various locations within a lesion so as to ensure a representative sampling of tissue.

It would be therefore advantageous to develop biopsy tools that have improved capabilities that address these identified needs. Namely, it would be advantageous to develop a biopsy, or tissue treatment tool that retrieves biopsy samples from or otherwise treats tissue in a variety of locations within a lesion simultaneously. It would also be advantageous to develop a biopsy tool that retrieves an adequate tissue sampling without requiring the removal of the LG from the EWC.

### SUMMARY OF THE INVENTION

According to the present invention, there is provided an endoscopic tool removably attached to a locatable guide, the tool comprising: a distal tool portion; and an attachment portion proximal of the tool portion; the locatable guide comprising a sensor being trackable in real-time; wherein said attachment portion is configured to removably mate with a distal tip of the locatable guide whereby the spatial relationship between the tool and the locatable guide is fixable.

The present invention represents a step forward in endoscopic biopsy and treatment procedures by providing tools designed for use with a three-dimensional locating system. The tools of the present invention are constructed to obviate the need for multiple navigation steps when taking one or more samples from a biopsy site, or performing one or more treatment procedures.

One aspect of the present invention provides a tool that attaches to the end of an LG. Such a device provides many advantages. First, a small, attachable tool presents a cost savings when compared to a more traditional tool long enough to be routed through a catheter. Typically, LGs and biopsy tools are single-use disposable devices. Reducing the material and complexity of a disposable is always preferred in order to reduce costs. Second, because the attachable tool is attached to the LG, the LG maintains its functionality and, therefore, can be used to monitor the location of the tool throughout a procedure. Moreover, the locatable tool may be used to take samples from or treat tissue in multiple locations in a lesion without retracting the tool and repositioning the EWC. Also, because the tool is not removed from the lungs between samples, a single tool may be used. Third, input from the LG may be used to map and display the places in the lesion where samples have been taken.

### BRIEF DESCRIPTION OF THE DRAWINGS

Figure 1 is a perspective view of an embodiment of a device of the present invention;
Figure 2 is a perspective view of another embodiment of a device of the present invention;
Figure 3 is a perspective view of an embodiment of a device of the present invention;
Figure 4 is a perspective view of an embodiment of a device of the present invention; and,
Figure 5 is a perspective view of an embodiment of a device of the present invention.

### DETAILED DESCRIPTION OF THE INVENTION

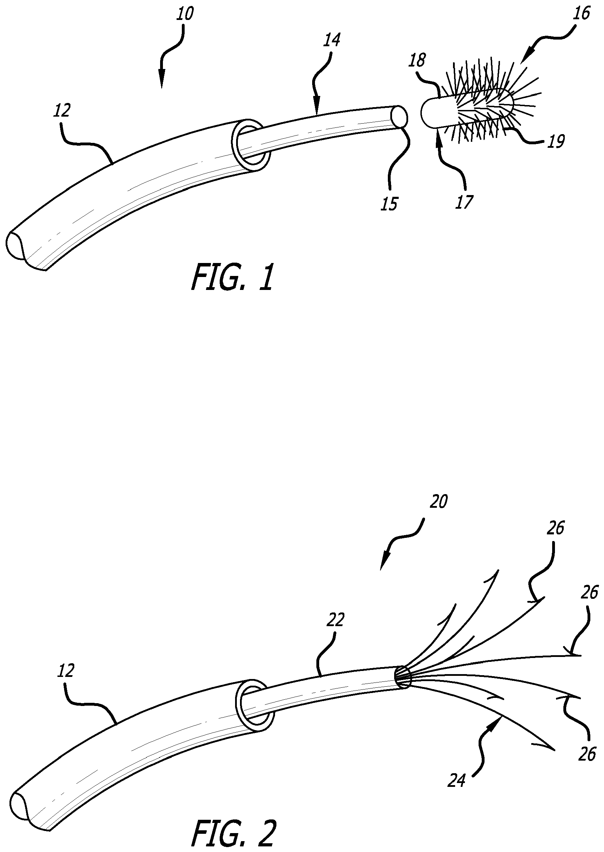

Referring now to Figure 1, there is shown an embodiment of a device 10 of the present invention. The device 10 generally comprises a delivery catheter or EWC 12, an LG 14, and an attachable tool 16. The tool 16 is shown as a brush, but may be take any form of any device. In this sense, Figure 1 is showing the general concept of an attachable tool 16, as well as providing a specific embodiment of a tool, in this case, a biopsy brush. Addressing the specific embodiment of a brush, the bristles of brush 16 may be configured with barbs or other tissue snagging devices if it is desired to increase the amount of tissue retrieved by the device 10.

Referring to the general embodiment of a generic tool, tool 16 has a proximal attachment portion 17 that is attachable to, or configured to mate with, the distal end 15 of the LG 14 by an end user, such as a physician or physician's assistant. In preferred embodiments, the attachment portion 17 includes a proximal skirt 18 that is sized to fit over the distal end 15 of the LG 14. The distal end 15 of the LG is configured to removably mate with the tool 16. For example, distal end 15 may include a snap connector, a threaded connector, a luer lock, etc. Similarly, the skirt 18 may be configured appropriately to accept whatever configuration is formed on the distal tip 15 of the LG 14. As shown, the distal tip 15 serves as a male component while the skirt 18 serves as a female component. One skilled in the art will understand that the distal tip 15 of the LG 14 may be configured as a female component for accepting a corresponding male component of the biopsy tool 16.

In a preferred embodiment, the LG 14 is a standard LG and the tool 16 is available in a variety of forms. Hence, a physician is able to attach any of several tools 16 to the distal end 15 of a standard LG 14 using a removable adhesive. Each of the various embodiments of tools described herein generally include a tool portion and an attachment portion. In the example shown in Figure 1, the tool 16 has an attachment portion 17, described above as a proximal skirt 18, and a tool portion 19, in this case a biopsy brush.

Another non-claimed example of a device 20 is shown in Figure 2. Like the device 10 of Figure 1, the device 20 is deliverable through a catheter or EWC 12. The device 20 further includes a biopsy tool 24 that radiates out of a sheath 22 when the tool 24 is advanced through the sheath 22. This biopsy tool 24 allows several samples to be taken simultaneously, covering a relatively large area. The biopsy tool 24 includes a plurality of tines 26, each of which is shown in the figure as being equipped with a barb for catching tissue.

It is envisioned that the sheath 22 may comprise an LG that has been modified to include a lumen for accommodating the biopsy tool 24. The device 20 is designed such that, after the tissue samples are taken, the biopsy tool is partially retracted into the sheath 22 until the tines 24 are brought together. The tines 24 and the sheath 22 may then be retracted into the EWC 12. Leaving the tines 24 partially extended prevents the loss of the tissue samples taken.

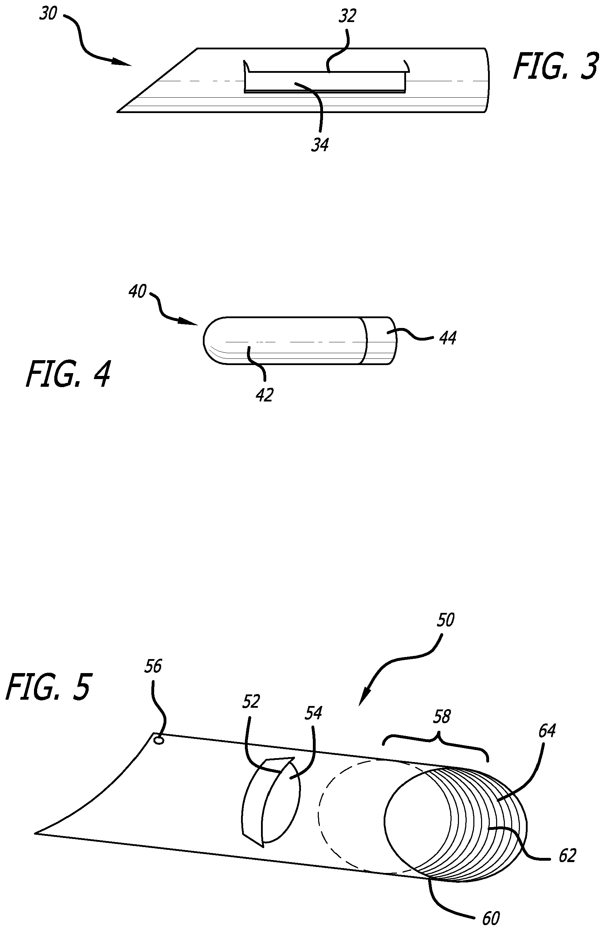

Figures 3 and 5 show various embodiments of tools according to the present invention. It is understood that all of these tools are attachable to the distal end of an LG in any of the various manners described above.

Referring first to Figure 3, there is shown a biopsy needle 30 according to the present invention. Biopsy needle 30 includes a scalloping blade 32 on a side surface of the needle 30. The scalloping blade allows a relatively large sample of tissue to be excised without the need for a jaw mechanism. The needle 30 also includes a hollow interior cavity 34 for receiving the tissue sample. In operation, the needle 30 is advanced into the target tissue and rotated. During rotation, the scallop blade 32 cuts a tissue sample and directs the sample into the cavity 34. Upon retraction, any remaining connection to the tissue is severed by a distal edge of the blade 32. Advantageously, little damage is done to any tissue that may lie between the targeted area, and the body lumen through which the LG was navigated to the site. Similarly, none of this tissue is sampled, as may be the case when using a brush device. Because the needle is rigid, the needle itself may easily be displayed using the navigation system, as the spatial relationship between the needle and the LG sensor is fixed.

Figure 4 shows a non-claimed seed implantation tool 40, including a seed 42 and a detachable coupling 44. The detachable coupling allows the marker seed 42 to be detached from the distal end of the LG once the marker seed 42 is inserted into a target location. The detachable coupling may be electrolytic, dissolvable, meltable, threaded, shaped-memory metal, stress-induced martensite, or any other known detachment mechanism used in percutaneously-delivered devices. The seed 42 may be a marker seed or a therapeutic seed. Many seeds acceptable for this purpose are shown and described in U.S. Publication 2009/0240140 entitled "Target Identification Tool for Intra-Body Localization". Nonlimiting examples include seeds adapted for use in marking locations, locatable visually or using ultrasound, Geiger meters, radio receivers, fluoroscopes, etc; or therapeutic seeds designed to administer drugs, chemotherapy, radiation therapy, cryo-therapy, ablation energy, or the like.

Figure 5 shows a biopsy needle 50 according to the present invention. Biopsy needle 50 includes a scalloping blade 52 on a side surface of the needle 50, oriented so the blade faces a proximal direction. The scalloping blade allows a relatively large sample of tissue to be excised without the need for a jaw mechanism. The needle 50 also includes a hollow interior cavity 54 for receiving the tissue sample. In operation, the needle 50 is advanced into the target tissue and retracted. During retraction, the scallop blade 52 cuts a tissue sample and directs the sample into the cavity 54. An air escape port 56 near a distal end of the needle 50 allows air or fluid to escape from the cavity 54, to more easily allow tissue to fill the cavity. Because the needle is rigid, the needle itself may easily be displayed using the navigation system, as the spatial relationship between the needle and the LG sensor is fixed. Figure 5 is oriented to show the attachment portion 58. It is understood that the attachment portion 58 of Figure 5 is representative of an attachment portion usable with any of the embodiments described herein. The attachment portion 58 includes a skirt 60 defining a female receptacle 62. The skirt 60, in this embodiment is shown with an interior surface containing threads 64. As state above, the threads may be replaced with any appropriate fastening device, including, but not limited to, luer lock, snap fit, friction fit, etc., and may be supplemented with an adhesive. It is also envisioned that the device 50 of the present invention may be designed to take tissue samples at the distal end of the device. As shown, the device includes a distal tip shaped like a scoop. An opening (not shown) to the cavity 54 could be provided. In this case, the scallop blade 52 would be omitted and the air escape port 56 would be located in a proximal location.

## Claims

1. An endoscopic biopsy tool (16) removably attached to a locatable guide (14), the tool comprising:
a distal tool portion (19); and
an attachment portion (17) proximal of the tool portion;
the locatable guide (14) comprising a sensor being trackable in real-time;
wherein said attachment portion is configured to removably mate with a distal tip (15) of the locatable guide whereby the spatial relationship between the tool and the locatable guide is fixable.

2. The endoscopic tool of claim 1 wherein said attachment portion comprises a skirt (18, 60) defining an opening into which a distal tip of a locatable guide may be inserted.

3. The endoscopic tool of claim 2 wherein said skirt comprises an inner surface defining threads (64) configured to mate with threads formed on a distal tip of a locatable guide.

4. The endoscopic tool of claim 1 wherein said distal tool portion comprises a biopsy brush.

5. The endoscopic tool of claim 1 wherein said distal tool portion comprises biopsy needle (30, 50).

6. The endoscopic tool of claim 5 wherein said biopsy needle comprises a pointed distal end and a scallop blade (32, 52) extending from a side surface of said needle, said scallop blade usable to excise tissue and direct excised tissue into a an opening that leads to an inner cavity (34, 54) of said biopsy needle.

7. The endoscopic tool of claim 6 wherein said scallop blade extends in a proximal direction, and/or wherein said scallop blade extends in a radial direction.

## Patentansprüche

1. Endoskopisches Biopsiewerkzeug (16), das entfernbar an einer lokalisierbaren Führung (14) befestigt ist, wobei das Werkzeug umfasst:
einen distalen Werkzeugabschnitt (19); und
einen Befestigungsabschnitt (17), der sich proximal von dem Werkzeugabschnitt befindet;
wobei die lokalisierbare Führung (14) einen Sensor umfasst, der in Echtzeit nachverfolgbar ist;
wobei der Befestigungsabschnitt dafür ausgelegt ist, mit einer distalen Spitze (15) der lokalisierbaren Führung entfernbar zusammenzupassen, wobei die räumliche Beziehung zwischen dem Werkzeug und der lokalisierbaren Führung fixierbar ist.

2. Endoskopisches Werkzeug nach Anspruch 1, wobei der Befestigungsabschnitt eine Einfassung (18, 60) umfasst, die eine Öffnung definiert, in die eine distale Spitze einer lokalisierbaren Führung eingeführt werden kann.

3. Endoskopisches Werkzeug nach Anspruch 2, wobei die Einfassung eine innere Oberfläche umfasst, die Gewinde (64) definiert, die dafür ausgelegt sind, mit Gewinden, die auf einer distalen Spitze einer lokalisierbaren Führung gebildet sind, zusammenzupassen.

4. Endoskopisches Werkzeug nach Anspruch 1, wobei der distale Werkzeugabschnitt eine Biopsiebürste umfasst.

5. Endoskopisches Werkzeug nach Anspruch 1, wobei der distale Werkzeugabschnitt eine Biopsienadel (30, 50) umfasst.

6. Endoskopisches Werkzeug nach Anspruch 5, wobei die Biopsienadel ein spitzes distales Ende und eine bogenförmige Schneide (32, 52), die sich von einer Seitenoberfläche der Nadel erstreckt, umfasst, wobei die bogenförmige Schneide verwendbar ist, um Gewebe herauszuschneiden und um herausgeschnittenes Gewebe in eine Öffnung, die in einen inneren Hohlraum (34, 54) der Biopsienadel führt, zu lenken.

7. Endoskopisches Werkzeug nach Anspruch 6, wobei sich die bogenförmige Schneide in einer proximalen Richtung erstreckt und/oder wobei sich die bogenförmige Schneide in einer radialen Richtung erstreckt.

## Revendications

1. Outil de biopsie endoscopique (16) fixé de manière amovible à un guide localisable (14), l'outil comprenant :
une partie d'outil distale (19) ; et
une partie de fixation (17) proximale par rapport à la partie d'outil ;
le guide localisable (14) comprenant un capteur qui peut être suivi en temps réel ;
dans lequel ladite partie de fixation est configurée pour se coupler de manière amovible à une pointe distale (15) du guide localisable en sorte que la relation spatiale entre l'outil et le guide localisable puisse être fixée.

2. Outil endoscopique selon la revendication 1, dans lequel ladite partie de fixation comprend une jupe (18, 60) définissant une ouverture dans laquelle une pointe distale d'un guide localisable peut être insérée.

3. Outil endoscopique selon la revendication 2, dans lequel ladite jupe comprend une surface interne définissant des filets (64) configurés pour se coupler à des filets formés sur une pointe distale d'un guide localisable.

4. Outil endoscopique selon la revendication 1, dans lequel ladite partie d'outil distale comprend une brosse de biopsie.

5. Outil endoscopique selon la revendication 1, dans lequel ladite partie d'outil distale comprend une aiguille de biopsie (30, 50).

6. Outil endoscopique selon la revendication 5, dans lequel ladite aiguille de biopsie comprend une extrémité distale pointue et une lame crantée (32, 52) s'étendant d'une surface latérale de ladite aiguille, ladite lame crantée étant utilisable pour exciser le tissu et diriger le tissu excisé dans une ouverture qui mène à une cavité interne (34, 54) de ladite aiguille de biopsie.

7. Outil endoscopique selon la revendication 6, dans lequel ladite lame crantée s'étend dans une direction proximale et/ou dans lequel ladite lame crantée s'étend dans une direction radiale.
